# EUROPEAN PATENT APPLICATION

(11) **EP 2 826 499 A1**
(43) Date of publication of application: **21.01.2015**
(21) Application number: 13760730.5
(22) Date of filing: 13.03.2013
(51) Int. Cl.: A61M 1/02

(54) **CONTAINER FOR TESTING BLOOD AND BLOOD SAMPLING INSTRUMENT**

(30) Priority: 14.03.2012 JP 2012057503
(71) Applicant: Terumo Kabushiki Kaisha, Tokyo 151-0072 (JP)
(72) Inventor: HAYAKAWA, Aya, Fujinomiya-shi Shizuoka 418-0004 (JP); AKIYAMA, Masahiro, Fujinomiya-shi Shizuoka 418-0004 (JP)
(74) Representative: TBK
(86) International application number: PCT/JP2013/057101
(87) International publication number: WO 2013/137361

(57) **Abstract**

A test blood container (1) includes a container body part (2) including an internal space (3) configured to store initial flow blood used for a test, a blood inflow port (5) configured to let the initial flow blood inflow, a blood outflow port (6) configured to let the stored initial flow blood outflow, an exhausting part (7) including a hydrophobic bacteria-impermeable filter (12) and configured to exhaust air inside the internal space (3), and a blocking part (8) arranged to the exhausting part (7) and configured to make it possible to block introduction of air into the internal space (3).

## Description

### Technical Field

The present invention relates to a test blood container configured to collect (store) initial flow blood to be tested and a blood collecting instrument including the test blood container.

### Background Art

Usually, as described, for example, in Patent Literature 1, a blood collecting instrument includes a vein puncture needle, a donor bag to house the blood collected through the vein puncture needle, plastic tubing to couple the vein puncture needle and the donor bag, a tubing segment which branches off in a midway from the plastic tubing, and a container (test blood container) to communicate with the plastic tubing through the tubing segment.

When the blood collecting instrument of Patent Literature 1 is used, initial flow blood of a donor (blood donor) is collected (inflow) into an internal chamber of a test blood container from an inlet port arranged to a lower part of the test blood container, before blood is collected (stored) from the donor into a donor bag, that is, before a main collection of blood is started. Then, the collected (stored) initial flow blood is collected (outflow) into a blood sampling vial, which communicates with an outlet port arranged to an upper part of the test blood container, and is used for various tests.

Therefore, in the blood collecting instrument of Patent Literature 1, initial flow blood used for various tests can be easily collected (stored). Also, even when bacteria which exist on or under skin are mixed into blood, the blood contaminated with the bacteria can be removed as the initial flow blood into an initial flow blood bag, and thus, the blood collected (stored) into a donor bag can be prevented from being contaminated with the bacteria.

### Citation List

### Patent Literature

Patent Literature 1: JP 2003-505185 A

### Summary of Invention

### Technical Problem

However, in the blood collecting instrument of Patent Literature 1, a worker (collecting person) starts collecting initial flow blood into a test blood container and visually checks, by liquid level of the initial flow blood, that a predetermined amount of the initial flow blood is collected into the container. Then the worker closes a clamp provided to a tubing segment and stops (complete) the inflow of the initial flow blood into the test blood container. As described, in the blood collecting instrument of Patent Literature 1, the inflow of the initial flow blood is stopped manually, whereby timing to close the clamp is different among the workers. Thus, variation in the amount of the initial flow blood collected (stored) into the test blood container is caused. The initial flow blood may be collected too much, or the minimum collecting amount thereof may not be secured. When the blood is collected too much, health damage of a donor and loss of the remaining blood are caused. Also, when the minimum collecting amount cannot be secured, various tests cannot be performed by using the initial flow blood, and thus, it is not possible to ensure the safety of the blood collected into the donor bag. Especially, when the minimum collecting amount cannot be secured, it is not possible to produce blood products from the blood collected through the blood collecting instrument, and thus, diminution of the blood products may be caused.

Also, in the blood collecting instrument of Patent Literature 1, air in plastic tubing and a tubing segment inflows into the test blood container before the inflow of the initial flow blood into the test blood container. Since the blood collecting instrument of Patent Literature 1 does not include means to exhaust the air inside the container, the air remains in the container when the inflow of the initial flow blood is completed. Thus, in the blood collecting instrument of Patent Literature 1, when the initial flow blood outflows into a blood sampling vial (testing instrument) while the test blood container, as a whole, is placed horizontally (in horizontally placed state) with a surface thereof touching a work table or the like, an outlet port is opened to an air remaining region in the container. Thus, the air is entrained into the initial flow blood which outflows from the outlet port. When such air entrainment is caused during the outflow of the initial flow blood, the amount of the initial flow blood collected into the testing instrument decreases, and thus, it is not possible to perform various tests by using the initial flow blood, similarly to what has been described above.

Accordingly, the present invention has been made to solve these problems. An object of the present invention is to provide a test blood container and a blood collecting instrument including the test blood container, with which it is possible to prevent variation in the amount of initial flow blood collected into the container, to secure the minimum collecting amount, and to prevent air entrainment during outflow of the initial blood into a testing instrument.

### Solution to Problem

To solve the problems above, a test blood container according to the present invention includes: a container body part including an internal space configured to store initial flow blood used for a test; a blood inflow port configured to communicate with the internal space to let the initial flow blood inflow; a blood outflow port configured to communicate with the internal space to let the stored initial flow blood outflow; an exhausting part including a hydrophobic bacteria-impermeable filter and configured to communicate with the internal space to exhaust air inside the internal space; and a blocking part arranged to the exhausting part and configured to make it possible to block introduction of air into the internal space.

According to the configuration above, since the exhausting part including a hydrophobic bacteria-impermeable filter is included, in both cases where the test blood container is used while being suspended from a stand or the like and being in a pendulous state and where the test blood container is used in a horizontally placed state, air gathered in an upper part of the internal space along with the inflow of the initial flow blood is exhausted from the exhausting part through the hydrophobic bacteria-impermeable filter. Then, the inflow of the initial flow blood automatically stops and the inflow is completed, when the initial flow blood touches the hydrophobic bacteria-impermeable filter. The internal space is filled only with the initial flow blood, and there is no remaining air. Also, since the bacteria-impermeable filter is hydrophobic, when the test blood container is in the horizontally placed state and in a case where the initial flow blood touches the bacteria-impermeable filter before the inflow of the initial flow blood is completed, air inside an internal space of the filter and the initial flow blood change places. Thus, an air block is not caused and the air is exhausted precedingly. Also, since the blocking part is included, it is possible to block air introduced from the outside (atmosphere) through the hydrophobic bacteria-impermeable filter of the exhausting part. Thus, air entrainment into the initial flow blood which outflows from the blood outflow port into a testing instrument can be prevented.

In addition, in the test blood container, the blood inflow port and the exhausting part are arranged to an end on the same side of the container body part. Also, the blocking part makes it possible to block the introduction of the air in the exhausting part and to block the inflow of the initial flow blood in the blood inflow part simultaneously.

According to the configuration above, the blocking part blocks the inflow of the initial flow blood in the blood inflow port, whereby the initial flow blood stored into the container body part through the blood inflow port can be prevented from flowing back into the blood collecting instrument, when the initial flow blood outflows into the testing instrument

Also, in the test blood container, the exhausting part includes a deformable part configured to be deformed by pressure from the blocking part to block the introduction of the air.

According to the configuration above, since the exhausting part includes the deformable part, the air introduced from the outside (atmosphere) through the hydrophobic bacteria-impermeable filter can be easily blocked by the pressure from the blocking part. Also, liquid level of the initial flow blood, which increases in the exhausting part, becomes stable by including a hard material especially on the side which includes the bacteria-impermeable filter, excluding the deformable part. Thus, it becomes easier to check a touch to the bacteria-impermeable filter by the initial flow blood.

In addition, the test blood container further includes a blood collecting part configured to communicate with the blood outflow part to collect the initial flow blood. The blood collecting part includes a needle assembly including a hollow needle configured to communicate with the blood outflow port, and a tubular holder including the needle assembly on one end and an opening on the other end, into the opening the testing instrument configured to house the initial flow blood being inserted.

According to the configuration above, since the blood collecting part is included, the initial flow blood stored in the container body part can easily outflow, through the needle assembly, into the testing instrument (such as pressure-reduced blood collecting tube) inserted into the holder.

The blood collecting instrument according to the present invention includes: a blood collecting needle; a storing part configured to house blood collected through the blood collecting needle; a blood collecting line configured to couple the blood collecting needle and the storing part; a branch line which branches off in a midway from the blood collecting line; and the test blood container configured to communicate with the blood collecting line through the branch line.

According to the configuration above, since the test blood container is included, the inflow of the initial flow blood automatically stops while the internal space is filled only with the initial flow blood and without any remaining air. In addition, the air entrainment during the outflow of the stored initial flow blood into the test instrument can be prevented.

Also, in the blood collecting instrument, the storing part is a blood bag or a blood separator.

Since the storing part is a blood bag or a blood separator, the blood collecting instrument can be used as a blood bag system or an apheresis collection set.

### Advantageous Effects of Invention

According to the test blood container and the blood collecting instrument of the present invention, variation in the amount of initial flow blood collected into the container is not caused and the minimum collecting amount can be secured. In addition, even when the initial flow blood outflows into a testing instrument, air entrainment is not caused.

### Brief Description of Drawings

Fig. 1 is a sectional view illustrating a configuration of an embodiment of a test blood container.
Fig. 2 (a) to Fig. 2 (c) are side views illustrating examples of a blocking part illustrated in Fig. 1.
Fig. 3 is a schematic view illustrating a configuration of an embodiment of a blood collecting instrument.

### Description of Embodiments

Embodiments of a test blood container according to the present invention will be described in detail with reference to the drawings.

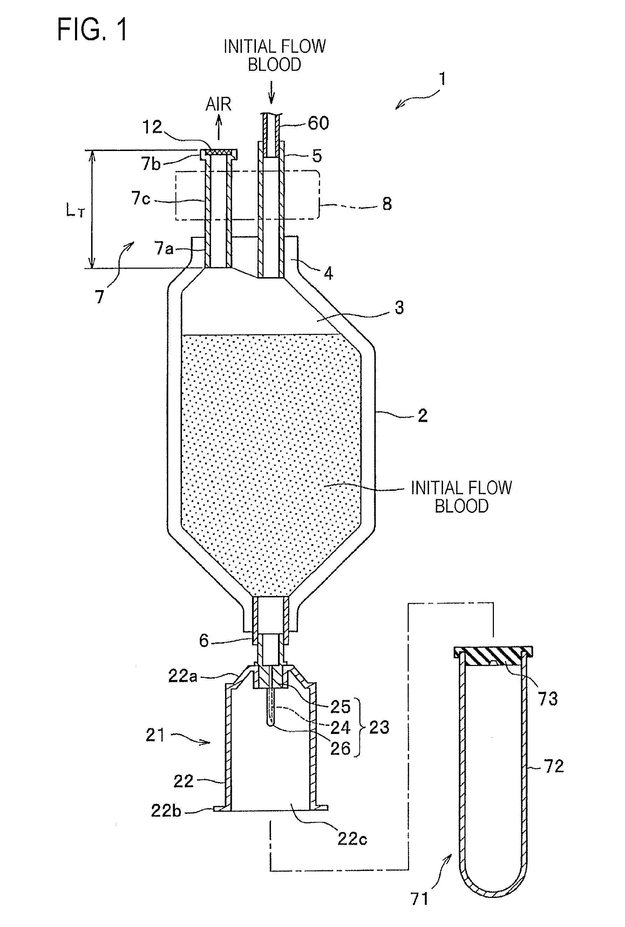

As illustrated in Fig. 1, the test blood container 1 includes a container body part 2, a blood inflow port 5, a blood outflow port 6, and an exhausting part 7. In the exhausting part 7, a blocking part 8 is arranged.

The container body part 2 includes, inside thereof, an internal space 3 configured to store (collect) initial flow blood used for a test. Preferably, the container body part 2 is bag-shaped and formed by laminating sheet materials including soft resin such as polyvinyl chloride and by forming a sealed part 4 in a peripheral edge thereof by fusion welding (such as thermal fusion welding and high-frequency fusion welding) or gluing. The internal space 3 is set to a size in which a previously set amount of blood (such as 25 ml) can be housed.

At ends of the container body part 2, the blood inflow port 5, the blood outflow port 6, and the exhausting part 7 are arranged to communicate with the internal space 3. When the test blood container 1 is suspended from a stand or the like and is in a pendulous state, the blood inflow port 5 and the exhausting part 7 are arranged on an upper end side of the container body part 2 and the blood outflow port 6 is arranged on a lower end side of the container body part 2.

Arrangements of the blood inflow port 5, the blood outflow port 6, and the exhausting part 7 are not limited to the arrangements in Fig. 1. Although it is not illustrated, for example, when the test blood container 1 is used in the pendulous state, the blood inflow port 5 may be arranged on the lower end side of the container body part 2, and the blood outflow port 6 may be arranged on the upper end side of the container body part 2, as long as the exhausting part 7 is arranged on the upper end side of the container body part 2. Also, when the test blood container 1 is used in the horizontally placed state, the blood outflow port 6 may be arranged to an end, on the same side of the blood inflow port 5, of the container body part 2, and the exhausting part 7 may be arranged to an end, on the opposite side of the blood inflow port 5, of the container body part 2. Note that the horizontally placed state means that the test blood container 1, as a whole, is placed horizontally in such a manner that a surface thereof touches a work table or the like.

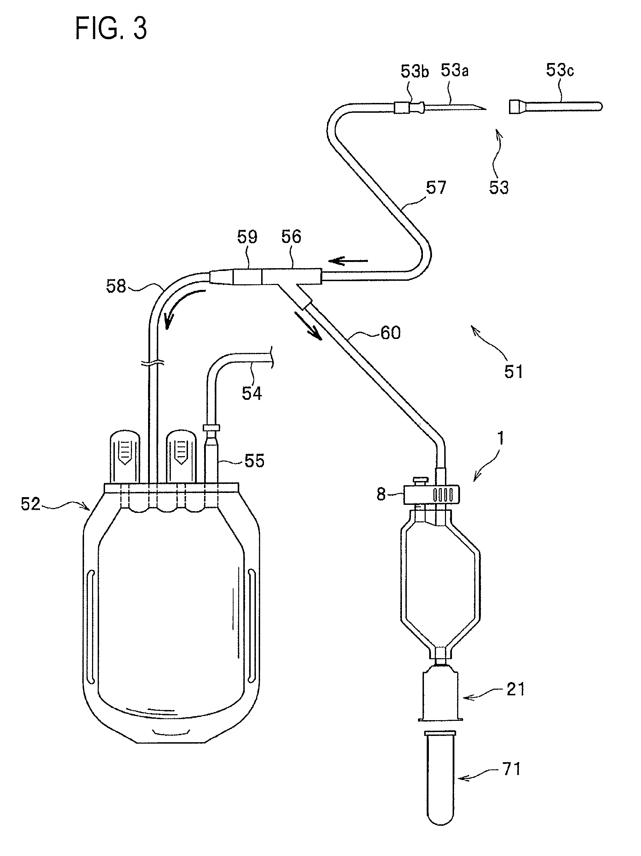

The blood inflow port 5 is a tubular body (tube) including soft resin such as polyvinyl chloride and is configured to communicate with the internal space 3 of the container body part 2 to let the initial flow blood inflow. During the preparation of the container body part 2, one end of the blood inflow port 5 is held between the sheet materials and is joined to the sealed part 4 of the container body part 2, by fusion welding or gluing, in such a manner to communicate with the internal space 3. Also, the other end of the blood inflow port 5 is joined to a branch tube 60 of the blood collecting instrument 51 by fusion welding or gluing (see Fig. 3).

The blood outflow port 6 is a tubular body (tube) including soft resin such as polyvinyl chloride and is configured to communicate with the internal space 3 of the container body part 2 to let the initial flow blood stored in the internal space 3 outflow into a testing instrument or the like. During the preparation of the container body part 2, one end of the blood outflow port 6 is held between the sheet materials and is joined to the sealed part 4 of the container body part 2, by fusion welding or gluing, in such a manner to communicate with the internal space 3. The other end of the blood outflow port 6 is joined to a blood collecting part 21, which will be described later, by fusion welding or gluing. Note that, although it is not illustrated, one end side of a hub 25 of a needle assembly 23 (blood collecting part 21), which will be described later, may be used as the blood outflow port 6, and the tubular body (tube) described above may not be used.

The exhausting part 7 is formed with a tubular body (tube) including, for example, hard or soft synthetic resin, and includes a hydrophobic bacteria-impermeable filter 12. The exhausting part 7 is configured to communicate with the internal space 3 of the container body part 2 to exhaust air inside the internal space 3. Also, the exhausting part (tube) 7 preferably includes a deformable part 7c configured to be deformed, by pressure from the blocking part 8 which will be described later, to block introduction of air. The deformable part 7c preferably includes a soft tube. Although it is not illustrated, for example, the exhausting part (tube) 7 includes a connection tube in which a soft tube is arranged on the side of one end 7a including the deformable part 7c and a hard tube is arranged on the side of the other end 7b, and the soft tube and the hard tube are connected to each other by fusion welding or gluing. The soft tube includes a material, such as polyvinyl chloride, which is the same as the container body part 2 and can be easily fusion welded or glued and the hard tube includes polycarbonate or the like. Note that the tube preferably includes a transparent material with which a touch to the bacteria-impermeable filter 12 by the initial flow blood can be easily checked.

During the preparation of the container body part 2, one end 7a of the exhausting part 7 is held between the sheet materials and joined to the sealed part 4 of the container body part 2, by fusion welding or gluing, in such a manner to communicate with the internal space 3. The other end 7b of the exhausting part 7 is opened to the outside (atmosphere). Also, the hydrophobic bacteria-impermeable filter 12 is preferably arranged to the other end 7b of the exhausting part 7.

Since the exhausting part 7 including the hydrophobic bacteria-impermeable filter 12 is included, air gathered in an upper part of the internal space 3 along with the inflow of the initial flow blood, is exhausted from the exhausting part 7 to the outside (atmosphere) through the hydrophobic bacteria-impermeable filter 12. Then, the inflow of the initial flow blood automatically stops and the inflow is completed, when the initial flow blood touches the hydrophobic bacteria-impermeable filter 12. The internal space 3 is filled only with initial flow blood and without any remaining air. As a result, compared to the conventional art in which the inflow is manually stopped, variation in the amount of the initial flow blood is not caused, and the minimum collecting amount can be secured. In addition, air entrainment is not caused during the outflow of the initial flow blood from the blood outflow port 6 into the testing instrument (pressure-reduced blood collecting tube 71).

To make the effect in the exhausting part 7 efficient, the hydrophobic bacteria-impermeable filter 12 is preferably a hydrophobic porous body, hydrophobic non-woven fabric, or the like having the strength to withstand venous pressure. Here, since the bacteria-impermeable filter 12 is hydrophobic, gas (air) permeates the bacteria-impermeable filter 12 but liquid (initial flow blood) does not permeate the bacteria-impermeable filter 12. Thus, in a case where the test blood container 1 is used in the pendulous state or is used in the horizontally placed state, the inflow of the initial flow blood automatically stops when the initial flow blood touches the bacteria-impermeable filter 12. Also, although it is not illustrated, when the test blood container 1 is in a horizontally placed state and in a case where the initial flow blood touches the bacteria-impermeable filter 12 before the inflow of the initial flow blood is completed, air inside an internal space of the filter and the initial flow blood change places, since the bacteria-impermeable filter is hydrophobic. Thus, an air block is not caused and the air is exhausted precedingly. As a result, there is no remaining air in the internal space. Examples of the bacteria-impermeable filter 12 is a porous body, non-woven fabric or the like, including a hydrophobic material such as polyolefin. Note that the pore diameter of the bacteria-impermeable filter 12 is preferably 0.01 to 100 µm. When the pore diameter is smaller than 0.01 µm, air permeability may not be enough. When the pore diameter is larger than 100 µm, it becomes difficult to control the permeation of bacteria.

The tube length L_{T} of the exhausting part 7 is preferably equal to or longer than 20 mm. By making the tube length L_{T} of the exhausting part 7 long, attachment of the blocking part 8, which will be described later, becomes easy and blocking of the introduction of the air can be easily performed with the blocking part 8. Note that the upper limit of the tube length L_{T} is not particularly limited, but when the tube length L_{T} of the exhausting part 7 is too long, it becomes difficult to handle the test blood container 1. Thus, the tube length L_{T} is preferably equal to or shorter than 70 mm.

The blocking part 8 is arranged to the exhausting part 7 and is a blocking member configured to make it possible to block the introduction of the air into the internal space 3 through the hydrophobic bacteria-impermeable filter 12 of the exhausting part 7 after a predetermined amount of the initial flow blood is stored into the internal space 3 of the container body part 2. Preferably, the blocking part 8 is a blocking member to make it possible, simultaneously, to block the introduction of the air in the exhausting part and to block the inflow of the initial flow blood in the blood inflow port 5 arranged to the end, on the same side of the exhausting part 7, of the container body part 2.

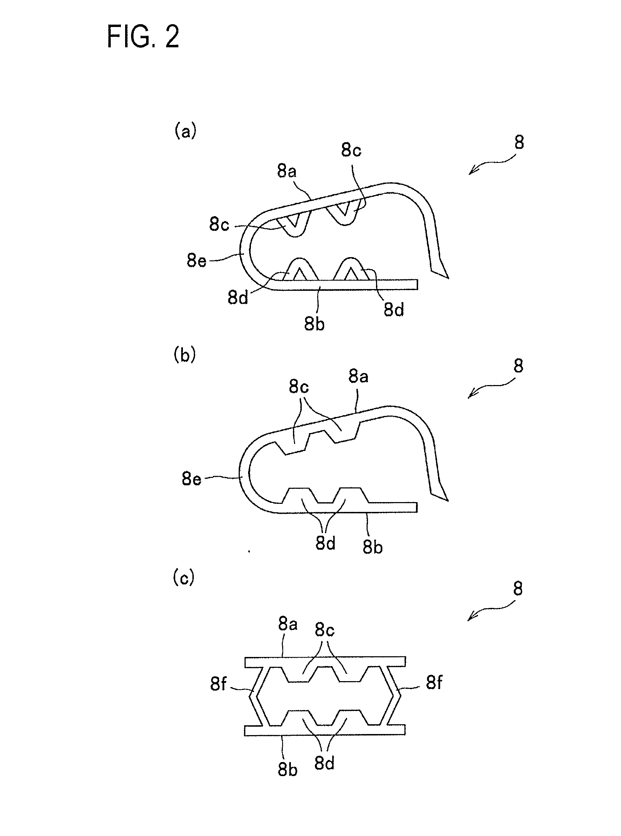

Such a blocking part 8 is preferably a clamp which blocks a tube of the air of the exhausting part 7 (tube), and which simultaneously makes it possible to block a blood tube of the blood inflow port 5 (tube), the clamp making it possible to block (occlude) a tube by pressure. For example, as illustrated in Fig. 2(a), the blocking part 8 is preferably a clamp which includes a first holding part 8a including two protruded first pressing parts 8c and a second holding part 8b including two second pressing parts 8d. One end part of the first holding part 8a and one end part of the second holding part 8b are coupled to each other via a curved part 8e in such a manner that the first pressing parts 8c and the second pressing parts 8d are arranged in opposing positions. In such a clamp, a tube (exhausting part 7 and blood inflow port 5), which is not illustrated, is held between the first holding part 8a and the second holding part 8b and pressed with the first pressing parts 8c and the second pressing parts 8d, whereby the introduction of the air and the inflow of the initial flow blood are blocked. Note that as illustrated in Fig. 2(b), the blocking part 8 may be a clamp in which pressing surfaces, to press the held tube, of the first pressing part 8c and the second pressing part 8d include flat surfaces. Also, as illustrated in Fig. 2(c), the blocking part 8 may be a clamp in which the first holding part 8a and the second holding part 8b are coupled to each other at both ends thereof via two curved parts 8f and 8f. Note that although it is not illustrated, the blocking part 8 may include a lock mechanism which keeps the blocked (occluded) state of the tube, the tube being blocked (occluded) by the pressure from the first pressing parts 8c and the second pressing parts 8d. Also, although it is not illustrated, the blocking part 8 may include one first pressing part 8c and one second pressing part 8d and may block only the exhausting part 7 (tube). In addition, the blocking part 8 is not limited to a clamp, but may be a cap or the like to cover the other end, opened to the outside (atmosphere), of the exhausting part 7, as long as the introduction of the air in the exhausting part 7 can be blocked.

Since such a blocking part 8 is included, the air introduced from the outside (atmosphere) through the hydrophobic bacteria-impermeable filter 12 of the exhausting part 7 can be blocked. Thus, the air is not introduced into the initial flow blood stored in the internal space 3 through the exhausting part 7, after the inflow of the initial flow blood is completed. As a result, the air entrainment into the initial flow blood, which outflows from the blood outflow port 6 into a pressure-reduced blood collecting tube 71 (testing instrument), can be prevented. In addition, since the blocking part 8 blocks the inflow of the initial flow blood in the blood inflow port 5, when the initial flow blood outflows into the testing instrument, the initial flow blood stored into the container body part 2 through the blood inflow port 5 can be prevented from flowing back into a blood collecting instrument 51 (see Fig. 3).

Next, other embodiments of the test blood container will be described with reference to the drawings.

As illustrated in Fig. 1, in addition to the configuration above, the test blood container 1 further includes a blood collecting part 21. The blood collecting part 21 communicates with the blood outflow port 6 and makes the pressure-reduced blood collecting tube 71 (testing instrument) collect the initial flow blood stored in the container body part 2.

The blood collecting part 21 includes a needle assembly 23, and a holder 22. The needle assembly 23 includes a hollow needle 24, a hub 25, and a rubber sheath 26. The hollow needle 24 includes a sharp needle tip at a leading end thereof and is formed with metal, hard resin, or the like. The hub 25 is fixed to a base end part of the hollow needle 24 and includes polyolefin or the like. The rubber sheath 26 covers the hollow needle 24. The hub 25 is joined to the blood outflow port 6, whereby the hollow needle 24 communicates with the blood outflow port 6. The holder 22 is a tubular member including polyolefin or the like, and includes the needle assembly 23 on one end 22a and an opening part 22c, into which the pressure-reduced blood collecting tube 71 (testing instrument) is inserted, on the other end 22b. The one end 22a of the holder 22 is placed on the side of an outer periphery of the hollow needle 24 of the needle assembly 23, and is joined to the hub 25 concentrically with the hollow needle 24.

Next, embodiments of the blood collecting instrument according to the present invention will be described.

As illustrated in Fig. 3, the blood collecting instrument 51 (blood bag system) includes a blood collecting needle 53, a blood collecting bag 52 (storing part and blood bag) configured to house the blood collected through the blood collecting needle 53, a blood collecting line configured to couple the blood collecting needle 53 and the blood collecting bag 52, a branch line which branches off in a midway from the blood collecting line, and the test blood container 1 configured to communicate with the blood collecting line through the branch line to collect (store) the initial flow blood. In the following, each of the configurations will be described. Note that the test blood container 1 has been described above, and thus, description thereof will be omitted.

The blood collecting needle 53 includes a hollow needle 53a, a hub 53b, and a protector 53c. The hollow needle 53a includes a sharp needle tip at a leading end thereof and is formed with metal, hard resin, or the like. The hub 53b is fixed to a base end part of the hollow needle 53a and includes polyolefin or the like. The protector 53c includes hard resin or the like and covers the hollow needle 53a.

The blood collecting bag 52 is a bag-shaped container formed by laminating resin sheets including polyvinyl chloride or the like, a peripheral edge thereof being fusion welded or glued. Inside the blood collecting bag 52, as described later in a blood treatment method, blood, in which the initial flow blood is removed (collected) from the blood collected through the blood collecting needle 53, is housed. Also, preferably, anticoagulant is previously housed inside the blood collecting bag 52.

One end of a tube 54 is connected to an upper part of the blood collecting bag 52 through a sealing member 55. On the other end of the tube 54, although it is not illustrated, a white blood cells removing filter, and a blood bag such as a collection bag (red blood cell collecting bag), a blood plasma collecting bag, and a bag including red blood cells preservation solution, are connected. Note that a conventionally well-known filter is used as the white blood cells removing filter. Also, the collection bag, the blood plasma collecting bag, and the bag including red blood cells preservation solution are bag-shaped containers including polyvinyl chloride or the like, similarly to the blood collecting bag 52.

In addition, one end of a tube 58 is connected to the upper part of the blood collecting bag 52. The other end of the tube 58 is connected to a branch connector 56, and the branch connector 56 is connected to the blood collecting needle 53 (hub 53b) through a tube 57. Therefore, the blood collecting line is formed by the tubes 57 and 58, and the branch connector 56. The blood collecting bag 52 and the blood collecting needle 53 are coupled to each other via the blood collecting line. Also, to the branch connector 56, the other end of the branch tube 60, one end of which is connected to the test blood container 1, is connected to form the branch line which branches off in a midway from the blood collecting line. Through the branch line, the test blood container 1 communicates with the blood collecting line. Also, between the branch connector 56 and the tube 58, a sealing member 59 is provided to prevent the initial flow blood from inflowing into the tube 58 on the side of the blood collecting bag 52 when the initial flow blood is collected (stored) into the test blood container 1 through the branch tube 60. Moreover, a clamp 61 is provided to the branch tube 60 to occlude a blood collecting tube of the initial flow blood.

Note that each of the sealing members 55 and 59 includes a short tube and a tubular body (not illustrated) which includes a solid leading end part housed inside the short tube to occlude the tube. Each of the sealing members 55 and 59 is a member to open a path inside the short tube by breaking the tubular solid leading end part. Also, the short tube includes a soft material such as polyvinyl chloride, and the tubular body includes a hard material such as polycarbonate. Note that the branch connector 56, tubes 54, 57, and 58, and the branch tube 60 include polyvinyl chloride or the like.

Next, a blood treatment method using the test blood container and the blood collecting instrument, which have been described above, will be described with reference to Fig. 3. Note that in respect to the configuration of the test blood container 1, Fig. 1 and Fig. 2(a) to Fig. 2(c) will be referred to appropriately.

First, as illustrated in Fig. 3, in the blood collecting instrument 51, the blocking part 8 (clamp) of the test blood container 1 is opened to make a tube of the blood inflow port 5 open. A blood collecting tube of the tube 58 is occluded by the sealing member 59. Note that the test blood container 1 is suspended from a stand or the like and is in a pendulous state, and the exhausting part 7 is placed at an upper end of the container body part 2. Also, the test blood container 1, as a whole, may be placed horizontally (in horizontally placed state) in such a manner that a surface thereof touches a work table or the like (not illustrated).

In such a state, the blood collecting needle 53 punctures a vein of a donor. Thus, the initial flow blood flows through the blood collecting needle 53, the tube 57, and the branch connector 56, and inflows into the branch tube 60, and then, collected (stored) into the test blood container 1. In this case, the blood collecting tube of the tube 58 is blocked by the sealing member 59, as described above. Therefore, the initial flow blood reliably flows into the branch tube 60 from the tube 57 through the branch connector 56. The air inside the tube 57, the branch connector 56, and the branch tube 60 is exhausted into the test blood container 1 (container body part 2) before the inflow of the initial flow blood.

In the test blood container 1, the initial flow blood inflows from the blood inflow port 5 into the internal space 3 of the container body part 2. Along with the inflow of the initial flow blood, the air inside the internal space 3 is gathered in an upper part thereof and exhausted to the outside (atmosphere) through the hydrophobic bacteria-impermeable filter 12 of the exhausting part 7. Then, after it is visually checked that the initial flow blood touches the hydrophobic bacteria-impermeable filter 12 and the inflow of the initial flow blood automatically stops, tubes of the exhausting part 7 (tube) and the blood inflow port 5 (tube) are blocked (occluded) with the blocking part 8, to block the introduction of the air, which is in the outside (atmosphere), from the exhausting part 7 and to block (stop) the inflow of the initial flow blood from the blood inflow port 5,simultaneously. Note that when the blocking part 8 is configured to block only the exhausting part 7, a clamp (not illustrated) provided to the branch tube 60 is closed to stop the inflow of the initial flow blood from the blood inflow port 5.

Then, collection of blood (main collection of blood) into the blood collecting bag 52 is started.

In this case, by breaking the solid leading end part (not illustrated) of the sealing member 59, a tube in the sealing member 59 is opened. With this operation, the blood collecting tube of the tube 58 is opened, and thus, the tube 57 and the tube 58 communicate with each other. Thus, the collected blood inflows into the blood collecting bag 52 through the tube 57, the branch connector 56, the sealing member 59, and the tube 58.

Also, along with the collection of the blood into the blood collecting bag 52, when determined to be possible by condition of the donor or condition of the collection of the blood, a sample is taken from the initial flow blood, which is collected (stored) into the test blood container 1, into a testing instrument such as the pressure-reduced blood collecting tube 71.

To take the sample, as illustrated in Fig. 1, the pressure-reduced blood collecting tube 71 is inserted into the blood collecting part 21 (holder 22) and is pushed into the innermost part of the holder 22, whereby the hollow needle 24 punctures and pierces through a rubber plug 73 fitted into a blood collecting tube body 72 of the pressure-reduced blood collecting tube 71. Thus, the initial flow blood stored into the test blood container 1 is sucked into the blood collecting tube body 72 and is taken as the sample. Then, the pressure-reduced blood collecting tube 71 is removed from the blood collecting part 21 (holder 22). Note that when the initial flow blood is taken as the sample into a plurality of pressure-reduced blood collecting tubes 71, this operation is performed repeatedly.

Note that as illustrated in Fig. 3, in the collection of blood into the blood collecting bag 52, after a previously set amount of blood is collected into the blood collecting bag 52, the blood collecting needle 53 is removed from the vein of the donor and, if necessary, the tube 58 and the branch tube 60 are occluded and sealed with a tube sealer or the like. Then, the test blood container 1 and the blood collecting needle 53 are uncoupled. As a result, the blood collecting bag 52 housing the blood, in which the initial flow blood is removed, can be obtained.

Although it is not illustrated, the blood housed in the blood collecting bag 52 is passed through the white blood cells removing filter to separate white blood cells and platelets, and the remaining blood components are collected into the collection bag. Then, the blood collecting bag 52 and the white blood cells removing filter are uncoupled. Then, the blood in the collection bag is centrifuged and separated into a red blood cell layer and a blood plasma layer. After the blood plasma is transferred into a blood plasma bag, the red blood cells preservation solution, which is in the bag including the red blood cells preservation solution, is added to and mixed with concentrated red blood cells remaining in the collection bag.

In the above, embodiments of the test blood container and the blood collecting instrument according to the present invention have been described, but the present invention is not limited to the embodiments. For example, in the blood collecting instrument according to the present invention, instead of the blood bag, a blood separator (such as centrifugal separator and membrane separator) may be provided as the storing part. That is, the blood collecting instrument is not limited to the blood bag system, but may be an apheresis collection set.

### Reference Signs List

- 1: test blood container
- 2: container body part
- 3: internal space
- 5: blood inflow port
- 6: blood outflow port
- 7: exhausting part
- 8: blocking part
- 12: bacteria-impermeable filter
- 51: blood collecting instrument
- 52: blood collecting bag (storing part)
- 53: blood collecting needle

## Claims

1. A test blood container comprising:
a container body part including an internal space configured to store initial flow blood used for a test;
a blood inflow port configured to communicate with the internal space to let the initial flow blood inflow;
a blood outflow port configured to communicate with the internal space to let the stored initial flow blood outflow;
an exhausting part including a hydrophobic bacteria-impermeable filter and configured to communicate with the internal space to exhaust air inside the internal space; and
a blocking part arranged to the exhausting part and configured to make it possible to block introduction of air into the internal space.

2. The test blood container according to claim 1, wherein the blood inflow port and the exhausting part are arranged to an end on a same side of the container body part, and the blocking part makes it possible to block the introduction of the air in the exhausting part and to block the inflow of the initial flow blood in the blood inflow port simultaneously.

3. The test blood container according to claim 1, wherein the exhausting part includes a deformable part configured to be deformed by pressure from the blocking part to block the introduction of the air.

4. The test blood container according to claim 1, further comprising a blood collecting part configured to communicate with the blood outflow part to collect the initial flow blood,
wherein the blood collecting part includes a needle assembly including a hollow needle configured to communicate with the blood outflow port, and a tubular holder including the needle assembly on one end and an opening on the other end, into the opening a testing instrument configured to house the initial flow blood being inserted.

5. A blood collecting instrument comprising:
a blood collecting needle;
a storing part configured to house blood collected through the blood collecting needle;
a blood collecting line configured to couple the blood collecting needle and the storing part;
a branch line which branches off in a midway from the blood collecting line; and
the test blood container according to any one of claims 1 to 4, configured to communicate with the blood collecting line through the branch line.

6. The blood collecting instrument according to claim 5, wherein the storing part is a blood bag or a blood separator.
